(19) Europäisches Patentamt / European Patent Office / Office européen des brevets

(11) **EP 3 813 040 A1**

(12) **EUROPEAN PATENT APPLICATION**
published in accordance with Art. 153(4) EPC

(43) Date of publication:
**28.04.2021  Bulletin 2021/17**

(21) Application number: **19825555.6**

(22) Date of filing: **05.06.2019**

(51) Int Cl.:
***G09B 5/00*** *(2006.01)*

(86) International application number:
**PCT/IB2019/054682**

(87) International publication number:
**WO 2020/003028 (02.01.2020 Gazette 2020/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:  **25.06.2018  CO 18006503**

(71) Applicant: **Universidad Antonio Nariño Bogota (CO)**
(72) Inventors:
• **HERNÁNDEZ DUARTE, Andrés Ignacio**
  **Bogota (CO)**
• **GUTIÉRREZ SALAMANCA, Rafael María**
  **Bogota (CO)**
• **CALDERÓN CHAGUALA, José Amilkar**
  **Bogota (CO)**

(74) Representative: **López Camba, Emilia**
  **SILEX IP**
  **C/ Poeta Joan Maragall 9, Esc. Izq., 3º Izq.**
  **28020 Madrid (ES)**

(54) **METHOD FOR THE DIAGNOSIS, DEVELOPMENT AND INCREASE IN AN ACCELERATED MANNER OF SPORT OR GAME INTELLIGENCE**

(57)    The present invention relates to a method for the diagnosis, development and increase, in an accelerated manner, of cognitive abilities in humans, which uses neuropsychological tests to assess cognitive abilities, in particular those that constitute an individual's sport intelligence or game intelligence: attention, memory, decision-making, processing speed and executive functions. The method comprises a step of assessing cognitive abilities, also called the diagnostic step; a step of planning physical activities and training according to the guidelines of a sport or activity that an individual wishes to improve; a step of implementing, in which the individual performs the activities designed in the planning step; and a step of final assessment. In addition, the steps can be carried out iteratively to the satisfaction of the individual or of a third party.

FIG. 1

## Description

## TECHNOLOGICAL SECTOR

The present invention is within the technical and technological sector that covers the design and implementation of training plans for athletes and evaluation and improvement of cognitive abilities and processes.

## BACKGROUND OF THE INVENTION

The psychological and quantitative analysis of human motor and cognitive abilities is a relatively new scientific and technical branch and one where different advances and approaches have developed with the objective of measuring, differentiating and improving human capacity with respect to a point of reference or starting point with an objective to reach a particular goal, e. g. that the response speeds exceeds a regional average of 10%.

Within the classifications of cognitive abilities in human beings, several of them have been defined as those that have special importance and differentiation with respect to others, such as wakefulness, conscience, natural language, learning, decision making, executive functions, memory, motor coordination, perception, planning, problem solving, thinking, among others.

To evaluate the cognitive abilities of humans, different neuropsychological tests have been proposed; Within the diverse tests that can be found in the bibliography are the Wisconsin letters test, the Stroop effect test, the Rey-Osterrieth figure test, the TMT-A or TMT-B tracing test, the Iowa gambling game, among others.

In addition, in the technical and technological sector that covers the design and implementation of training plans for athletes and assessment and improvement of cognitive abilities and processes (above all there exists developments that focus on the training of isolated cognitive processes and in what refers to training devices and rehabilitation platforms, but it is common for these to focus on rehabilitating patients and not on improving the intelligence and reaction of a person who has not suffered an accident or illness, or more specifically, on a person whose performance wants to be improved to exercise a certain profession or activity, e.g. a sports activity.

Document WO2012148524(A1) discloses a medium by computer in which it is possible to simulate a sport stage with the purpose of evaluating the response of a user against continuous or interrupted sports stimulus and where it is possible to simulate the situations of different sports and receive entryways of information by the user with different methods of entry. However, in said document it is not possible to measure integral and quantitative values that determine in a concise manner the sports intelligence or gaming intelligence of the user; nor the possibility of evaluating the user without the need of resorting to simulation means.

Document US20160253917(A1) discloses a cognitive or multisensory simulation system for the training and improvement of athletes and other populations, that consists of a system of semantic language to transmit information and instructions to an individual in a confusing or normal manner and that permits measurements of cognitive processes in the individual based on their reactions to said stimulus, and when possible, between other additional activities, warn the individual of an incoming stimulus. However, said document also does not mention the possibility of measuring in an integral and quantitative way, the values that determine in a concise manner the sport intelligence or gaming intelligence of the user; nor the possibility of evaluating the user without the need to resort to simulation means.

The present invention provides a defined and structured method in terms of iterative activities and processes that allow to evaluate and improve the cognitive abilities relative to the areas of the sport, where the evaluation is used with the goal of objectively classifying a player in accordance with their gaming intelligence or sport intelligence. The present method represents advantages while it delimits guidelines to quantitatively evaluate the players and based on this, improve their abilities from a numerical starting point, while using techniques to measure cognitive abilities and physical trainings that are applied iteratively are used until a desired performance or improvement is reached. Furthermore, the present method preferably uses known and delimited neuropsychological tests, allowing its extensive replication and therefore also the comparison in the execution of the method, for this purpose also taken into account standardized regional averages according to the description of the present invention.

## BRIEF DESCRIPTION OF THE INVENTION

The invention corresponds to a method for the diagnosis, development and accelerated increment of the gaming intelligence in people, especially athletes, that is comprised of a series of iterative processes, in which neuropsychological tests are used, oriented towards the improvement of the different abilities that make up the gaming intelligence of a person. Where the iterative processes comprise at least one phase of the diagnosis or evaluation of cognitive abilities; a phase of the planning of physical activities and training according to the preestablished guidelines; one phase of implementation or application of development or training plan and; a phase of development, contemplating the possibility of eventual updates of each plan, thanks to the possibility of performing the phases iteratively to the satisfaction of the individual or third party.

In the context of this invention, gaming intelligence is defined as the integral evaluation and interrelation of five cognitive abilities: memory, attention, processing speed, decision making, and the executive functions, where the executive functions in turn are made up of cognitive skills such as initiative, motivation, consistency, self-control, cognitive flexibility, among others.

The initial phase of the diagnosis makes up the implementation of neuropsychological tests for the measurement of cognitive abilities of an individual, where said tests are selected from the Wisconsin letters test, the Stroop effect test, the Rey-Osterrieth figure test, the TMT-A or TMT-B tracing test, the Iowa gambling game, among others. Once the neuropsychological tests have been completed, and they have obtained their respective measurements, weighted values are associated to these measurements to obtain a single value according to a cognitive model that allows to quantifiably define the current state of the gaming intelligence of the individual.

Later, and based on the results obtained in the first phase, the design of the training plan proceeds, principally focused on strengthen and/or improve those/that cognitive ability (es) that are (is) found below the others regarding its quantitative measurement and that which have more influence on the gaming intelligence.

The tests that are part of the training plan are applied or trained to the people and can be modified by level of complexity such as, low, medium, and high, among others. These trainings can be intensified by the application of a greater number of tests and training in that cognitive ability that is below the others in terms of its quantitative measurement. These trainings are performed through periodic sessions which in turn perform with different frequencies, duration and intensity according to the objectives of each case, keeping in mind that the sessions with a high training level are preferably performed in the period immediately following a competition.

Finally, in the evaluation phase, quantitative measurements of the gaming intelligence are performed, with the objective of identifying the progress of each one of the abilities used. According to the obtained results, feedback is given with respect to the post-training state of the cognitive abilities of the individual and a re-planning of the sessions is carried out at different levels of complexity according to the new characteristics and the new relative deficiencies of the cognitive abilities of the individual.

## DESCIPTON OF THE FIGURES:

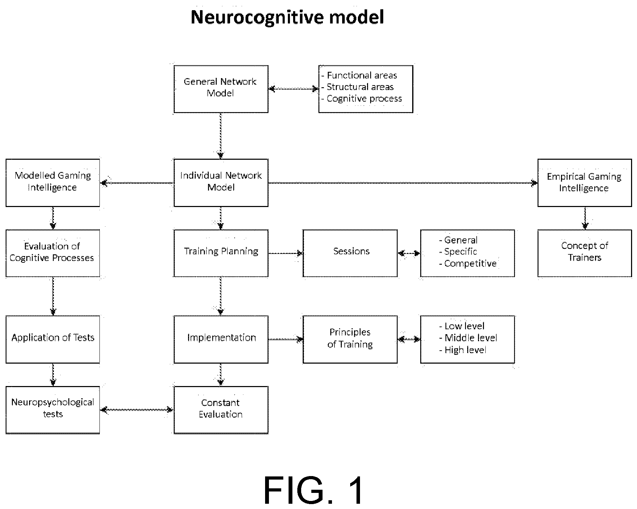

Figure 1 shows a conceptual map that describes the characteristics and general interrelations for the method of the present invention.

## DETAILED DESCRIPTION OF THE INVENTION

The present invention refers to a system and/or device, and also to a method for the diagnosis, development and accelerated increment of the sport intelligence or gaming intelligence in people, especially athletes, where in the method and system neurological tests are used for the measurement and improvement of the different characteristics that comprise the gaming intelligence of a person. Said method and system are supported by a neurological model that can be created from a relationship between any measurable neurological abilities and any of the identifiable human brain regions; And where said method and system comprises a series of iterative processes where it is possible to use neuropsychological tests to complement the improvement of the skills that constitute the gaming intelligence. Likewise, the iterative processes comprise a planning phase, also called diagnostic phase, a planning phase of physical activities and training, a phase of implementation with respect to these neuropsychological tests and regard to their related aspects within each training session, as well as a final evaluation and feedback phase.

In the context of this invention, gaming intelligence is defined as the capacity and its integral quantitative evaluation of a number of cognitive abilities, as well as the qualitative interrelation of said cognitive abilities, where preferably five of these cognitive abilities are evaluated, and where the cognitive abilities in general are related to different structural areas, or regions, of the human brain, where the five abilities that are taken into account preferably are: memory, attention, processing speed, decision making, and executive functioning; where the executive functioning in turn, comprises of cognitive abilities such as initiative, motivation, consistency, self-control, cognitive flexibility, among others. Where the concepts are defined in the present descriptive memory in order to give additional context to the invention, and where it is made explicit that the understanding of said concepts is not limited to what is described herein.

In general, memory corresponds to the capacity of an individual to acquire, code, store information, and retrieve information on knowledge, abilities, and experiences; attention corresponds to an individual's capacity to select from the environment those stimuli that are relevant for reaching a goal; processing speed corresponds to the speed of an individual to respond to a known stimuli but one which has not previously been automized mentally or muscularly by said person. Decision making corresponds to the capacity of the individual to select an alternative, with satisfactory results, from the point of view of a goal and within a range of existing options. Likewise, executive functions are the group of cognitive abilities that serve an individual to formulate goals and the planification, and execution of actions and activities for the achievement of said goals, executive functions comprise of initiative, motivation, consistency, self-control, cognitive flexibility, inhibition, anticipation, among others.

The structural areas, or regions of the human brain are defined according to the most recent consensus in terms of divisions of the human brain according to their functions or relations, for example, the lateral dorsal frontal region, the ventromedial frontal region, the posterior parietal lobe, the inferior temporal lobe, the angular gyrus, the ascending reticular formation, the amygdala, the cerebellum, among others. Within the framework of the present invention, these divisions of the human brain are not limited to knowledge and current consensus, but that they can be updated according to the advances of science in this sense, the former insofar as the method and systems described here use the current state of the art to create a neurological model that is described in greater detail in subsequent paragraphs.

The neurological model that is taken into account in the moment of implementing the method and system described here is created primarily from the language of graph theory, wherein a number of nodes is defined which is preferably equivalent to the total number of regions of the human brain identified in the current scientific consensus, and wherein each of the nodes corresponds to a specific and identifiable region of the human brain; Likewise, the number of cognitive skills to be considered based on the sport or human activity that one wishes to potentize is defined, preferably the five mentioned above: memory, attention, speed of processing, decision making, and the executive functions, and similarly to the definitions for the regions of the brain, current studies and current consensus of the scientific community are used to determine the relationship between the selected cognitive abilities and the regions of the human brain, each set is defined of relationships or links between regions of the brain, where each set of relationships or links is related to a cognitive ability, as a functional area of the brain.

In the definition of the model based on graph theory, there are a set of nodes (related to the regions of the brain) that are related through possible links between these nodes, where the existence of each of the possible links that connect any nodes i and j is defined as affirmative if there exists at least one cognitive ability that is related to the regions of the brain represented by nodes i and j, in accordance with the cognitive abilities and their relationships with the human brain, according to studies or a neurological consensus and decisions previously described in the present specification. The conjunction of existing nodes and links can be defined then as the graphic model of gaming intelligence. In the context of the present invention, the relationship of the nodes through links for the proposed neurological model is, in accordance with the mnemonics characteristic of graph theory, non-directional.

It is possible to relate the particular gaming intelligence of an individual person through the gaming intelligence graph model through the translation of their cognitive abilities to said gaming intelligence graph model, where the performance of the individual in terms of their cognitive abilities are expressed in the gaming intelligence graph model as the set of weights of existing links and optionally, by other measures that can be calculated based on the weight of each of the existing link or other characteristics of the individual's network for example, density, modularity, diameter of network, middle nodal grade, average weighted nodal grade, centrality, among others. The translation of the quantitative cognitive abilities of the neuropsychological tests implemented by weight values in links for the graph model is preferably performed by an algorithm comprising mathematic calculations and it is possible to implement by a computer, for the context of the present invention, this algorithm is called weight calculation algorithm.

Where the algorithm of calculation of weights just mentioned, by which the weights of the bonds between the nodes of the model of graph of an individual are obtained, is related to an algorithm that includes within its calculations the following equation for the obtaining of link weights based on measurements obtained with neuropsychological tests:

$$l_{ij} = H_1 \sum_{h=2}^{N} H_h$$

Where the subscripts I and J represent any pair of nodes denoted by i and j, and where the letter H represents the numerical value, preferably standardized according to what is said in subsequent paragraphs, assigned to a cognitive ability and in N represents the total number of cognitive abilities to use. Given that the cognitive abilities to use are preferably speed processing, memory, attention, decision-making and executive functions, the preferred value of N is five. The preferred value for the ability H with the subscript of 1 corresponds with the measured value for speed processing, and subsequently, the cognitive abilities denoted with H with subscripts from 2 to N correspond, each, to the measured value for the remaining cognitive abilities to use in the method of the present invention.

Whichever equation equivalent, and in general whichever equation that intends to calculate a value associated with each of the links is within the spirit of the present invention.

Density, in the context of graph theory, is defined by its most general form as the proportion of existing links with respect to possible links according to the quantity of nodes, being then the conscious of effective relationships R, with the relationships that, according to the number N of nodes, equals N(N-1) when the links are directional or at N(N-1)2 when the links are non-directional. In accordance with the neurological model whose construction is comprised by the method of the present invention, where the links that relate the nodes are characterized as being non-directional, the equation for density for a graph model G related with the present invention:

$$D(G) = \frac{2R}{N(N-1)}$$

Where it is also possible to evaluate the density of one or more subgraphs *subG<sub>i</sub>*, action for that which can define a subgraph *subG<sub>i</sub>* through the selection of one or more nodes that are part of a graph model G, together with the links of said selection of nodes.

Modularity, in the context of graph theory, aims to measure the structure of a net or graph, and is related to the propensity of a net to divide in modules, also called groups, groupings or communities; nets with high modularity are characterized by comprising solid connections between nodes of the same module, but scarce between nodes that are part of said module.

The diameter of a net is related to all the possible paths that can be created between the nodes; once all the minimal paths that relate each pair of nodes have been calculated, the diameter of the net is defined as the one with the greatest longitude of said minimal paths, or what is the same, the minimal path for the most separated nodes between each other inside the same net, where the separation of two nodes is usually determined for the quantity of links that separate these nodes.

The degree of each node is defined as the number of links connected to it, where each link can also relate itself with an associated weight allowing the subsequent definition of a degree by weight or weighted degree, which is related to a numerical value equivalent to the arithmetic sum of the weights of each link connected to a node. Subsequently, it is possible to define the middle degree and the average weighted degree for a net or subnet, by taking the degree average for the nodes and the average of the weighted degree for said nodes, respectively.

The centrality of a net or graph is related to the position, existing links, and the importance of its nodes, and it can be defined in different ways, one of which allows the medial centrality of a node j to be defined as the quantity of possible paths between all of the pairs of nodes in the net, that comprise of said node J in its travel. It is also possible to define the medial centrality as the average of said values for all the nodes in the net, among other statistical values.

In this way it is possible to define a quantitative game intelligence from the analysis of each performance of the particular graph model of an individual at a given moment, where the quantitative game intelligence is defined as the sum of a set of emergent qualities that are can measure the graph model, where the emergent qualities are selected from modularity, average weighted nodal grade, among others. Preferably density, modularity, network diameter and average weighted nodal grade. The mathematical equation that describes the above description is:

$$IJ = \sum_{i=0}^{N} M_i$$

Wherein: IJ denotes the quantitative gaming intelligence; the letter M with the subscript i denotes each one of the emergent qualities selected for the model according to the embodiment of the invention; and the letter i denotes a discreet variable that pertains to the interval of natural numbers denoted by (1,N), where N corresponds to the number of emergent qualities selected for the specific realization of the invention. The quantitative definition previously described allows to evaluate in a more objective manner, the performance or actual state of an individual, as well as the development in its performance and the comparison of qualitative values along the different iterations of the method and system, here exposed.

As it has previously been mentioned the present methods and system support, in different phases of its implementation, in the realization of neuropsychological tests with the goal of evaluating and potentiating the abilities of an individual, especially those of a soccer athlete. The neuropsychological tests are preferably but not limited to the Wisconsin card test, the Stroop effect test, the Rey-Osterrieth figure test, the TMT-A or TMT-B tracing test and the Iowa gambling test.

The Wisconsin card test allows to measure the capacity of one subject in terms of problem solving, and especially their capacity to modify strategies when faced with changes in the environment all of which through a test where the subject must demonstrate flexibility in the face of changing confirmation schemes. The test normally consists of 48 cards and 4 key cards, in the context of the present invention, the test preferably has said number of cards, but the scope of the invention allows for changes to the number of cards previously mentioned. In the application of the test the cards are revealed, one by one, to an individual who must associate them to one of the four cards that the individual finds face up (the key cards), the individual receives feedback from the examiner, which can be an informational tool, about whether their selection is correct according to the actual parameters of the test; once the individual completes a number of hits, which can be consecutive hits or non-consecutive hits according to the evaluator, the rules of association change and it is fed back keeping into consideration said change; the process is repeated as described above until the rules have changed six or more times or until the cards run out; all of the above while relevant information is taken regarding the performance of the subject in the test.

In the Stroop effect test three pages are preferably used, whether that be on paper or on a screen, or whatever other mode can translate images, each one with five columns, each one with twenty element, indicating to the subjects that they should pronounce out loud what they find written in an element or the color of the typography or glyph of each element; on the first page the elements correspond to words, or in general written language, that represent colors, in black typography; on the second page the elements correspond to whichever glyph or figure translated in a specific color; on the third page the elements correspond to words that represent colors, translated in typographies of different colors, where the discrepancy between typographic color and the color represented in written language; on the first two pages, the subject is asked to pronounce or make out the only color that its visually represented, for each element, on the third page, the subject is instructed to concentrate only on the color represented in written language or only on the visible color of the typography.

The Rey-Osterrieth complex figure test is done in order to evaluate the perceptual organization and the visual memory of an individual. During the test, the individual is asked to reproduce and complex picture, first by copying it, and then by solely using memory as support. Also, there exists preferably an interval of time after the individual makes the copy of the picture, and before the individual makes the copy using their memory, where said interval of time is invested in activities that are preferably not related to the figure or picture. Scoring is based on the precision on the reproduced picture.

The trace test, in the modalities TMT-A and TMT-B, consists of instructing an individual to connect the points of a graphic that contains an infinite number of elements, in one modality of the test the elements correspond to numbers, in another modality the elements correspond to written language characters, and in another possible modality a group that consists of numbers and characters in general written language. The time spent doing the task and the mistakes made while completing the task are measured.

The Iowa gambling task allows to classify the behavior of a subject in terms of their aversion or propensity of risk through an experiment where the participant must choose cards or letters in order to win or lose money, simulated in an indirect manner, the generality of scenarios of decision making in the real world.

Then, the method according to the invention is comprised of a series of stages, those which are described in detail below. The first stage consists in the diagnosis or implementation of neuropsychological tests to measure the cognitive abilities of an individual. This phase is aimed at determining the current state of the gaming intelligence of the individual through the application of neuropsychological tests and the analysis of the obtained information, from where the individuals state of intelligence is obtained, identifying strengths and weaknesses of the individual.

The tests are selected from neuropsychological tests mentioned and explained previously, and in general, from whatever neuropsychological test not mentioned. The neuropsychological tests are administered to the individuals from one of three levels of complexity, low medium, and high, where said tests allow for the evaluation of each cognitive ability associated to said neuropsychological tests, performing a greater number of tests and physical trainings in that cognitive skill that is found below the others in terms of its quantitative measurement; likewise said tests and trainings are performed through periodic sessions with trainings of high level performed preferably in the periods of time immediately before a competition.

In the context of the present invention, the levels of complexity of the training sessions and the neurological tests are defined as this: low level refers to simple exercises at different speeds, basic training for each process, in accordance with training principles, it is applied above all to general training; middle level refers to an increase in the complexity of exercises, at different speeds, it applies to above all pre-competitive training (prior to multi-player or multi-team competition); high level refers to activities carried out during a competition, game reality.

Once the neuropsychological test and their respective measurements have been performed, the results obtained through the association of numerical values of said measurements are analyzed to obtain an integral evaluation of the gaming intelligence, where said numerical values are special values obtained from a numerical transformation selected from transformation to percentile, transformation to type T score, among other statistical evaluation and comparison techniques; where the statistical transformation is preferably standardized according to the average values of the region from which the individual is born to evaluate and train, this way guaranteeing the standardization at the time to compare the advances at the time of applying the method to different individuals.

Preferably the numerical values obtained from the numerical transformations recently mentioned are used for the calculation of the weight of the links comprised by the neurological model that represent the structured areas of an individual, so to later calculate the quantitative gaming intelligence, according to what was previously described in the present descriptive memory.

From the gaming intelligence and the quantitative cognitive abilities of the individual, those that had a minor and major performance are identified, and those that represent important strengths or weaknesses within their performance. That which will be taken into account for the next stage of the method of the present invention.

After the diagnosis, the stages of the development plan for the gaming intelligence and its implementation continue. First, a training plan is developed that centralizes in potentiating the cognitive deficiencies or skills that are found below the others or optionally improve the desired abilities according to the preferred position of an athlete; for example in a soccer forward player, the improvement of processing speed and executive functioning skills take priority, while a goalkeeper prioritizes improvement in speed processing and attention skills. For the planning of activities, above all physical activities, selected training principles of continuity, individuality, progression, alternation, specificity, among others, are considered.

The implementation of the development plan of the gaming intelligence is based on the training that consists in a physical regimen that is adjusted to the conventional guidelines of the sport or activity that the individual practices or that activity in which the individual wishes to improve their performance. The physical training consists preferably in competitions against other players or athletes, according to the activity in which the individual wishes to improve their performance.

The activities carried out during said physical training are recorded by video camera, sensor, or another image or video capturing device; furthermore, the cognitive abilities, performance and evolution of the individual are registered and considered for future evaluations, where said evaluations are carried out with special relation to the cognitive abilities that comprise gaming intelligence. Said physical training can consist in one or various repeated sessions in one or more days, according to planning done by the training or supervisor of the tests.

Likewise, the method and the system preferably comprise of the implementation of a conjunction of complementary mental and physical activities that promote the development of the cognitive abilities such as individual or group activities selected from pyramid of cards, distribution of objects according to objective and memory, mnemonic memorization, sum of figures, ordering of numbers, board games, sudoku, crosswords, word searches with limited time, among others.

Finally, the method according to the invention, includes a final evaluation whose objective consists in providing evidence of the individual's progress with respect to the activity carried out, e.g. the sport they practice, and the method is carried out in a reiterative manner from the initial phase, where neuropsychological tests are newly carried out to analyze the progress of the individual as to the cognitive abilities that comprise the game intelligence, allowing to also carry out a re-planning of the physical training phase, or field training, taking into account in a special way the new relative deficiencies of the performance of an individual's cognitive abilities with respect to the performance evidenced by the rest of their cognitive abilities. In this way it continues until the individual or trainer is satisfied with the characteristics, abilities, or performance of the individual.

In other words, in the final evaluation of the gaming intelligence the techniques used in the initial diagnostic phase are used. Later, the quantitative results obtained by the individual in the initial phase of the method are compared according to the invention with the quantitative result of the present final evaluation, where the analysis of said results will allow to show the progress of the individual in the activity they carry out.

In all of the instances where the neuropsychological tests for the evaluation of the individual's abilities are carried out, it allows that said tests are used through the use of an electoral device with an interface that can interact with said individual; where said electronical device selects preferably from, but not limited to, a computer with a screen, virtual reality glasses, an electronic tablet, a smart phone, among others; where said electronic device allows for the automatic evaluation of the cognitive abilities through the use of neuropsychological tests; optionally, the tests are carried out without electronic devices: by the implementation of tests which the user must read and complete using pencil and paper or by the personal evaluation done by a psycho-technical supervisor, of the abilities of an individual while training doing physical exercises, preferably those of a sport and preferably those of soccer, or while said individual plays a competitive sports match, preferably of soccer. During the performance of any modality of neuropsychological tests, the complementary evaluation and the accompaniment by a psycho-technical supervisor can be used, who can be used in a complementary way to the individual and who can give guidelines for the tests. Figure 1 shows the in a general way the previously mentioned characteristics and the interrelations between some of the steps y the described concepts in the present descriptive memory.

In the modality of the invention where the neuropsychological tests are carried out through an electronic device, the individual can interact with said device through input elements selected from a keyboard, a mouse, a prompter, accelerometer, a camera, a motion sensor, a light sensor, joystick, rudder, in general whatever electronic device of input what whatever possible combination of said elements; likewise, the individual can interact and receive visual and auditory information among others, through a screen, speaker, an augmented reality viewer, in general whatever outgoing electronic device and whatever possible combination of said elements.

The present invention provides a defined and structured method in terms of activities and iterative processes that allow to evaluate and improve cognitive abilities relative to the areas of sport, where the evaluation is used with the purpose of objectively classifying a player in accordance with their gaming intelligence and sport intelligence. The present method represents advantages in such that it delimits guidelines to quantitatively evaluate the players and based on this, improve their abilities from a numerical standpoint while techniques of cognitive abilities and physical training are used, which are applied iteratively until a desired performance or improvement is reached. What's more, the present method uses preferably known and delimited neuropsychological tests, allowing their extensive replication and therefore also the comparison in the execution of the method, for the purpose to also consider standardized regional averages in accordance with the description of the current invention.

The system and/or device that is part of the present invention comprises a database, an information entry interface, an interface that allows the display of information, and a processor of information. Where the information entry interface can be physical or virtual, and likewise where the information display interface can be physical or virtual.

The database allows for the storage of the neurological model's information, corresponding to the nodes that represent the different regions of the brain and the existing links according to the definitions of the neurological consensus selected for the realization of the invention. Likewise, said database allows for the storage of characteristic information of each individual in terms of the magnitude or weight of the links of their particular graph model, and in general whatever information related with the previously described method and to the individuals involved as their individual and collective performance regarding the evaluation provided by the neuropsychological tests. The information processor can carry out procedures related with the weight calculation algorithm and in general the mathematical calculations related with the implementation of the method according to the invention.

The figures presented in this description correspond to merely illustrative purposes of the invention. It is implied that the described figures do not limit the scope of the invention. A person versed in the arts is capable of conceiving later modifications to the determined principles in the present document.

Even though some modalities of the invention are described in the present description, it will be appreciated that a number of modifications and other modalities can be conceived by those experts in the material subsequent to the disclosure of the present invention. For example, the characteristics described here can be applied in other modalities. Thus, it is understood that the attached claims are intended to cover all the modifications and modalities that are within the spirit and reach of the present description.

**Claims**

1. A method for the development and accelerated increment of an individual's gaming intelligence that comprises the following phases:

   a. a diagnostic phase that analyses an individual's different cognitive abilities that make up his or her gaming intelligence, selected from memory, attention, processing speed, decision making, and the executive functions through the application of neuropsychological tests.
   b. a planning phase of the development of the gaming intelligence in which a plan of activities, selected from physical and cognitive activities, individual and group, is drawn up according to the deficiencies in the intelligence of the individual's play.
   c. a phase of implementation of activities and training in accordance with the plan of activities developed in the previous phase; and,
   d. a final evaluation phase in which neuropsychological tests are applied and compared with the results of the diagnostic phase.

2. The method of claim 1 wherein the diagnostic phase comprises the analysis of the information obtained from the application of the neuropsychological tests using a model based on the theory of graphs and the identification of the individual's strengths and weaknesses regarding their gaming intelligence.

3. The method of claim 1 wherein, after the final evaluation phase, the planning phase, the execution phase, and the final evaluation phase are repeated many times until the desired esult is obtained.

4. The method of the previous claims in which neuropsychological tests are selected from the Wisconsin letters test, the Stroop effect test, the Rey-Osterrieth figure test, the TMT-A or TMT-B tracing test, and the Iowa gambling game, among others.

5. The method of the previous claims where the plan of physical and cognitive activities of the planning phase is selected according to the sport or activity in which that individual wants to improve his or her performance.

6. A system for the development and accelerated increase of the gaming intelligence of an individual comprising a database, an information input interface, an information display interface, and an information processor; where the database stores the corresponding information in a network model in which the nodes represent the different regions of the brain and the links between them.

7. The system for the development and accelerated increment of the gaming intelligence of an individual of claim 6 wherein the database stores the information of each individual corresponding to the amount, relationships, and weight of each link in the individual's network model.

8. The system for the development and accelerated increment of the gaming intelligence of an individual of claim 6 wherein the information processor processes and obtains information from the links of the network model based on the numerical information selected from the individual application of neuropsychological tests and the concept of a trainer versus the cognitive abilities an individual.

9. The system for the development and accelerated increment of the gaming intelligence of an individual of claim 6 wherein the information input interface is selected from keyboards, mouse, pointer, accelerometer, camera, motion sensors, light sensors, joysticks, rudders, among others and combinations of these.

10. The system for the development and accelerated increment of the gaming intelligence of an individual of claim 6 wherein the information display interface is selected from screens, speaker, augmented reality viewers, virtual reality viewers, among others and combinations of these.

## Neurocognitive model

FIG. 1

| **INTERNATIONAL SEARCH REPORT** | International application No. |
|---|---|
| | PCT/IB2019/054682 |

**A. CLASSIFICATION OF SUBJECT MATTER**

*G09B5/00* (2006.01)

According to International Patent Classification (IPC) or to both national classification and IPC

**B. FIELDS SEARCHED**

Minimum documentation searched (classification system followed by classification symbols)
G09B

Documentation searched other than minimum documentation to the extent that such documents are included in the fields searched

Electronic data base consulted during the international search (name of data base and, where practicable, search terms used)

EPODOC, INVENES

**C. DOCUMENTS CONSIDERED TO BE RELEVANT**

| Category* | Citation of document, with indication, where appropriate, of the relevant passages | Relevant to claim No. |
|---|---|---|
| X | US 2013330693 A1 (SADA JASON  ET AL.) 12/12/2013, Abstract, claims and Figures 29 and 30. | 1-10 |
| A | ES 2576456T T3 (RSI VIDEO TECH INC) 07/07/2016, claims and figures. | 1-10 |
| A | US 5035625 A (MUNSON GERALD L  ET AL.) 30/07/1991, the whole document. | 1-10 |
| A | WO 2009022024 A1 (LLOPIS LLOPIS JOSE DANIEL ET AL.) 19/02/2009, Pages 4-10. | 1-10 |
| A | ES 2337556T T3 (HEALTH DISCOVERY CORP) 27/04/2010, Page 7, line 50- Page 8, line 5. | 1-10 |

☐ Further documents are listed in the continuation of Box C.     ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| | "&" document member of the same patent family |

| Date of the actual completion of the international search | Date of mailing of the international search report |
|---|---|
| 15/10/2019 | **(16/10/2019)** |
| Name and mailing address of the ISA/ | Authorized officer |
| | G. Foncillas Garrido |
| OFICINA ESPAÑOLA DE PATENTES Y MARCAS | |
| Paseo de la Castellana, 75 - 28071 Madrid (España) | |
| Facsimile No.: 91 349 53 04 | Telephone No. 91 3493282 |

Form PCT/ISA/210 (second sheet) (January 2015)

| INTERNATIONAL SEARCH REPORT | | International application No. | |
|---|---|---|---|
| Information on patent family members | | PCT/IB2019/054682 | |

| Patent document cited in the search report | Publication date | Patent family member(s) | Publication date |
|---|---|---|---|
| US2013330693 A1 | 12.12.2013 | US2017294136 A1 | 12.10.2017 |
| | | US2017103670 A1 | 13.04.2017 |
| | | AU2016250358 A1 | 10.11.2016 |
| | | JP2014509892 A | 24.04.2014 |
| | | JP6366128B B2 | 01.08.2018 |
| | | CN103765491 A | 30.04.2014 |
| | | CA2830299 A1 | 01.11.2012 |
| | | AU2012249185 A1 | 19.09.2013 |
| | | EP2676256 A1 | 25.12.2013 |
| | | EP2676256 A4 | 27.01.2016 |
| | | WO2012148524 A1 | 01.11.2012 |
| ES2576456T T3 | 07.07.2016 | ES2677274T T3 | 31.07.2018 |
| | | US2016078734 A1 | 17.03.2016 |
| | | US9679455 B2 | 13.06.2017 |
| | | EP2911125 A1 | 26.08.2015 |
| | | EP2911125 B1 | 11.09.2019 |
| | | BRPI0709172 A2 | 28.06.2011 |
| | | BRPI0709172 B1 | 08.05.2018 |
| | | US2009179988 A1 | 16.07.2009 |
| | | US8081073 B2 | 20.12.2011 |
| | | US2009167862 A1 | 02.07.2009 |
| | | US9189934 B2 | 17.11.2015 |
| | | CA2647300 A1 | 08.05.2008 |
| | | CA2647300 C | 09.07.2013 |
| | | AU2007314584 A1 | 08.05.2008 |
| | | WO2008054479 A2 | 08.05.2008 |
| | | WO2008054479 A3 | 30.10.2008 |
| | | WO2007111802 A2 | 04.10.2007 |
| | | WO2007111802 A3 | 06.11.2008 |
| | | WO2007038184 A2 | 05.04.2007 |
| | | WO2007038184 A3 | 14.06.2007 |
| | | US2007257195 A1 | 08.11.2007 |
| | | US7463146 B2 | 09.12.2008 |
| | | US2007063840 A1 | 22.03.2007 |
| | | US7463145 B2 | 09.12.2008 |
| | | CA2623501 A1 | 05.04.2007 |
| | | CA2623501 C | 13.05.2014 |
| | | AU2006294985 A1 | 05.04.2007 |
| | | AU2006294985 A8 | 02.06.2011 |
| | | EP1927089 A2 | 04.06.2008 |
| | | EP1927089 A4 | 06.10.2010 |
| | | EP2011093 A2 | 07.01.2009 |
| | | EP2011093 A4 | 09.05.2012 |
| | | EP1999733 A2 | 10.12.2008 |
| | | EP1999733 A4 | 24.04.2013 |
| ES2337556T T3 | 27.04.2010 | AT450834T T | 15.12.2009 |
| | | US2006224539 A1 | 05.10.2006 |
| | | US7383237 B2 | 03.06.2008 |

Form PCT/ISA/210 (patent family annex) (January 2015)

<div align="center">

## INTERNATIONAL SEARCH REPORT

Information on patent family members

</div>

| International application No. |
|---|
| PCT/IB2019/054682 |

| Patent document cited in the search report | Publication date | Patent family member(s) | Publication date |
|---|---|---|---|
| | | US2002165837 A1 | 07.11.2002 |
| | | US6996549 B2 | 07.02.2006 |
| | | WO02059828 A2 | 01.08.2002 |
| | | WO02059828 A3 | 14.08.2003 |
| | | JP2004536367 A | 02.12.2004 |
| | | JP3947109B B2 | 18.07.2007 |
| | | EP1356421 A2 | 29.10.2003 |
| | | EP1356421 B1 | 02.12.2009 |
| | | CA2435290 A1 | 01.08.2002 |
| | | CA2435290 C | 01.05.2012 |
| | | AU2002243783B B2 | 19.07.2007 |
| WO2009022024 A1 | 19.02.2009 | CN102084380 A | 01.06.2011 |
| | | JP2010535370 A | 18.11.2010 |
| | | US2010268520 A1 | 21.10.2010 |
| | | EP2180434 A1 | 28.04.2010 |
| | | EP2180434 A4 | 06.07.2011 |
| US5035625 A | 30.07.1991 | WO9101540 A1 | 07.02.1991 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012148524 A1
- US 20160253917 A1